# EUROPÄISCHE PATENTSCHRIFT

(11) **EP 0 295 671 B1**
(45) Veröffentlichungstag und Bekanntmachung des Hinweises auf die Patenterteilung: **25.08.1993**
(21) Anmeldenummer: 88109598.8
(22) Anmeldetag: 16.06.1988
(51) Int. Cl.: A61M 16/01, A61M 11/00

(54) **Sicherheitsfüllvorrichtung zum Befüllen und Entleeren eines Narkosemittelverdunsters**
Safety filling device for filling and emptying an anesthetic vaporizer
Dispositif de sécurité pour le remplissage et la vidange d'un vaporisateur anésthésique

(30) Priorität: 19.06.1987 DE 3720326
(43) Veröffentlichungstag der Anmeldung: 21.12.1988
(73) Patentinhaber: Drägerwerk Aktiengesellschaft, 23542 Lübeck (DE)
(72) Erfinder: Mohr, Helmut, D-2406 Stockelsdorf (DE); Falb, Wolfgang, Dipl.-Ing., D-2061 Klein Wesenberg (DE); Wallroth, Carl-Friedrich, Dr., Dipl.-Ing., D-2400 Lübeck (DE)

(56) Entgegenhaltungen:
- DE-B- 1 566 592
- DE-B- 1 657 174
- DE-B- 1 900 271

## Beschreibung

Die Erfindung betrifft eine Sicherheitsfüllvorrichtung zum Befüllen und Entleeren eines Narkosemittelverdunsters über einen angeschlossenen Vorratsbehälter bestehend aus einem bewegbaren, rohrförmigen Leitungsstück, welches an seinen Enden mit einem Indexstück zum Eingriff in den Verdunster und mit einem Behälteranschlußstück versehen ist, wobei das rohrförmige Leitungsstück einen vom Füllkanal getrennten Belüftungskanal aufweist.

Zur Befüllung eines Narkosemittelverdunsters wird die Anwendung eines Sicherheitsfüllsystems vorgeschrieben, bei dem der Füllschlauch ein unverwechselbares, zugeordnetes Indexstück zum Eingriff in den Verdunster und eine kodierte Flaschenkappe zur ausschließlichen Verbindung mit einem Vorratsbehälter von bestimmter Füllung aufweist. Das rohrförmige Leitungsstück muß bei angeschlossener Narkosemittelflasche für den Füllvorgang angehoben und für das Entleeren abgesenkt werden.

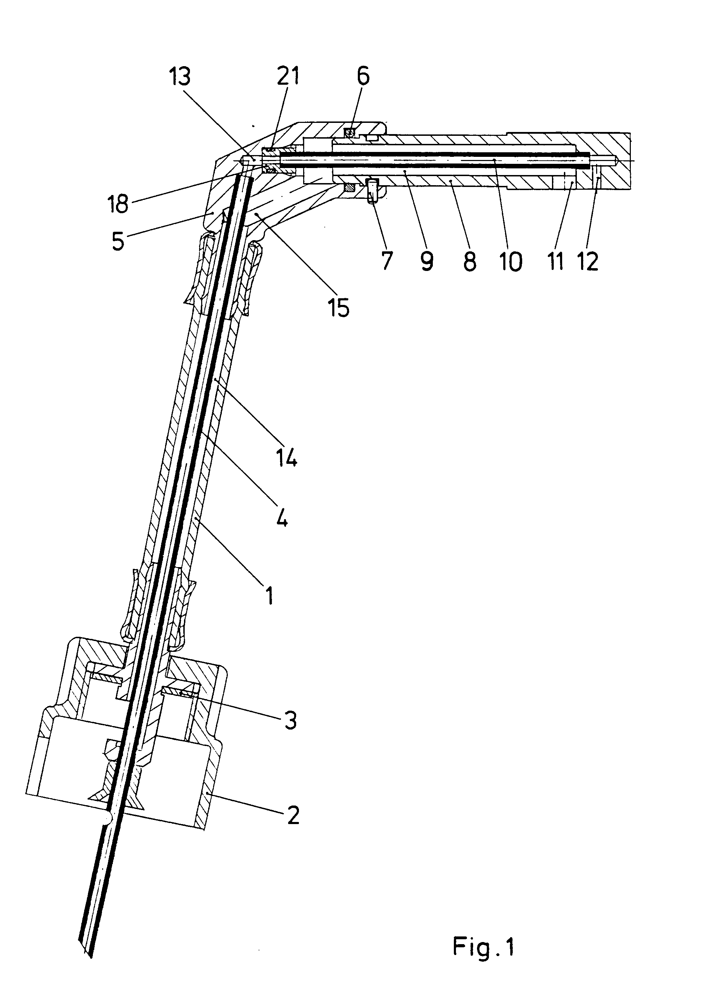

Bei der Anwendung rohrförmiger Leitungsstücke mit dem Merkmalen des Oberbegriffs des Anspruchs 1 in Form von elastischen Füllschläuchen, wie sie in der Drägerwerk-Gebrauchsanweisung 5327.09 vom Dezember 1984 Abb. 1 dargestellt sind, treten Biegebeanspruchungen auf, welche einen vorzeitigen Verschleiß und einen im Gebrauch gefährlichen Ermüdungsbruch an den Verbindungsstellen herbeiführen können. Außerdem ist es schwierig, den Füllschlauch während des Nichtgebrauchs zu verschließen, so daß ein Austreten der Narkosemittelflüssigkeit und der Dämpfe sicher verhindert werden. Eine weitere Schwierigkeit bei der Verwendung derartiger Füllschläuche besteht darin, daß elastische Kunststoffe Weichmacher enthalten müssen, die durch das Narkosemittel herausgelöst und in unzulässiger Weise in den Atemluftkreislauf von Patienten gelangen könnten.

Aus der DE-B 15 66 592 ist ein Narkosemittelverdunster mit einem schwenkbaren Verbindungsstück zum Ansatz eines Vorratsbehälters bekannt. Dieses Verbindungsstück enthält einen kodierten, nur zur vorgesehenen Flasche passenden Flaschenanschlußstutzen sowie ein Ventil, welches bei eingesetzter, das Narkosemittel enthaltender Flasche bzw. bei eingeführtem flexiblen Verbindungsstück geöffnet ist. Eine solche Vorrichtung erscheint hinsichtlich der Handhabung verbesserungsbedürftig.

Eine Vorrichtung zum Einfüllen einer Narkoseflüssigkeit aus einer Flasche in den Füllraum eines Narkosemittelverdampfers ist aus der DE-B-1 900 271 bekannt, bei welcher über einen flexiblen Schlauch ein Flüssigkeitskanal in den Füllraum mündet, aus dem ein Entlüftungskanal in den flexiblen Schlauch abgeht und in der Vorratsflasche mündet.

Weiterhin zeigt die DE-B-1 657 174 eine Sicherheitsfüllvorrichtung, die ein Anschlußstück umfaßt, über welches das flüssige Narkosemittel aus einem Vorratsbehälter in einen Narkosemittelverdunster eingebracht werden kann. Beim Verbinden des Anschlußstücks mit dem Verdunster werden Ventile geöffnet, die den Durchfluß des Narkosemittels aus dem Anschlußstück in den Vorratsbehälter freigeben. Nach Entfernen des Anschlußstückes werden die Ventile wieder geschlossen.

Zur Lösung der Aufgabenstellung ist bei einer Sicherheitsfüllvorrichtung der eingangs beschriebenen Art vorgesehen, daß am verdunsterseitigen Ende des rohrförmigen Leitungsstücks ein Gelenkstück angeschlossen ist, in dem ein absperrbares, Leitungsabschnitte von Füllkanal und Belüftungskanal aufnehmendes Indexstück drehbar gelagert ist. Durch das zwischen dem Gelenkstück und dem Indexstück gebildete Drehgelenk ist eine Biegebeanspruchung des rohrförmigen Leitungsstücks zum Heben und Senken während des Füllbzw. Entnahmevorgangs nicht erforderlich. Außerdem wird die Verwendung starrer metallener bzw. inflexibler, weichmacherfreier Kunststoffwerkstoffe ermöglicht. Bei Anwendung von formstarren Materialien lassen sich ferner konstante Querschnitte von Füllkanal und Belüftungskanal erzielen und batchunabhängige einheitliche Befüll- und Entleerzeiten für verschiedene gleichartige Sicherheitsfüllvorrichtungen erzielen.

Im Bereich des zwischen Indexstück und Gelenkstück gebildeten Drehgelenks kann vorteilhaft eine Absperrvorrichtung integriert sein, welche in der Absperrlage den Füllkanal und den Belüftungskanal abschließt. Diese läßt sich zweckmäßig derart bewegbar ausbilden, daß sich die Absperrvorrichtung beim Abnehmen des Indexstücks vom Verdunster in die Absperrlage bewegt. Die Absperrvorrichtung kann dabei sowohl durch axiales Verschieben als auch durch radiales Verdrehen des Indexstücks zwangsweise betätigt werden. Für bestimmte Anwendungszwecke erscheint jedoch auch die Anwendung eines handbetätigten Ventils vorteilhaft.

In einer günstigen Ausführung kann das Indexstück in dem Gelenkstück kolbenartig gegen eine Druckfeder verschiebbar gelagert sein und mit dem Füllkanal sowie mit dem Belüftungskanal nur in der Freigabelage kommunizierende, drehstellungsunabhängige Leitungsabschnitte aufweisen. Der Belüftungskanal kann dabei vorteilhaft als zentrales Leitungsstück ausgebildet sein, welches einseitig in das Indexstück und in einen kolbenartigen Führungsteil eingreift,wobei Indexstück und kolbenartiger Führungsteil als Baueinheit im Gelenkstück drehbar oder verschiebbar und drehbar gelagert sind.

Die drehstellungsunabhängige Leitungsverbindung kann zweckmäßig durch umfangsseitige Nutenausnehmungen am Indexstück und/oder am Führungsteil gebildet sein.

Das rohrförmige Leitungsstück läßt sich vorteilhaft aus einem äußeren Füllrohr aus Metall oder aus weichmacherfreiem, transparenten bzw. transluzentem Kunststoff und einem inneren Belüftungsrohr aus Metall oder einem anderen narkosemittelbeständigen Werkstoff zusammensetzen. Das Indexstück und das Gelenkstück sind dabei vernickelte Messingteile oder bestehen aus anderen narkosemittelbeständigen, korrosionsfreien Werkstoffen. Alternativ lassen sich diese Teile aus weichmacherfreiem Kunststoff, wie Polyamid, herstellen. Die Dichtungselemente zur Abdichtung zwischen dem Gelenkstück und dem in diesem drehbar gelagerten Indexstück mit Führungsteil, sind vorteilhaft narkosemittelbeständige Schnurringdichtungen, beispielsweise aus Fluor-Kautschuk.

Die Erfindung wird nachfolgend anhand von Ausführungsbeispielen näher erläutert; es zeigen:
- Fig. 1: einen Längsschnitt durch eine Sicherheitsfüllvorrichtung,
- Fig. 2: einen Querschnitt durch eine alternative Ausführung einer Sicherheitsfüllvorrichtung in der Absperrlage,
- Fig. 3: eine Ausschnittsdarstellung aus Fig. 2 in der Freigabelage.

In Fig. 1 ist ein rohrförmiges Leitungsstück 1 gezeigt, welches an seinem einen Ende mit einem als Schraubkappe 2 ausgebildeten Behälteranschlußstück versehen ist. In der Schraubkappe ist zur Abdichtung der einzuschraubenden in der Zeichnung nicht dargestellten Narkosemittelflasche eine Dichtungsscheibe 3 vorgesehen.

Das rohrförmige Leitungsstück 1 besteht aus weichmacherfreiem transparentem oder transluzentem Kunststoff oder aus Metall und nimmt in seinem Innenraum ein metallenes Belüftungsrohr 4 auf. Am anderen Ende des rohrförmigen Leitungsstücks 1 ist ein Gelenkstück 5 angeschlossen.

In dem Gelenkstück 5 befindet sich ein umfangsseitig mit einer Schnurringdichtung 6 abgedichtetes Indexstück 8, welches einen Leitungsabschnitt 9 des Füllkanals und einen zentralliegenden Leitungsabschnitt 10 des Belüftungskanals aufnimmt. Die Auslaßöffnungen 11,12 von Füllkanal und Belüftungskanal der Leitungsabschnitte 9,10 können zur Absperrung durch eine bei Gebrauch abnehmbare auf das Indexstück 8 aufgesetzte Abdichtkappe verschlossen sein.

Ein im Gelenkstück 5 befindlicher Stift 7 greift in eine Ringnut des Indexstücks 8 ein und sichert das Indexstück 8 gegen axiale Verschiebung im Gelenkstück 5.

Der Leitungsabschnitt 10 des Belüftungskanals steht unter Zwischenschaltung eines mit dem Indexstück 8 drehbaren, hohlen Führungsteils 18, der eine Schnurringdichtung 21 aufweist, über eine Verbindungsbohrung 13 mit dem Belüftungsrohr 4 in Verbindung.

Der in dem rohrförmigen Leitungsstück 1 gebildete Füllkanal 14 ist über einen in dem Gelenkstück 5 liegenden Verbindungsabschnitt 15 mit dem Leitungsabschnitt 9 des Füllkanals verbunden.

Bei an der Schraubkappe 2 angesetztem Narkosemittelbehälter wird das Indexstück 8 in die entsprechende Aufnahmeöffnung am Narkosemittelverdunster eingeführt. Durch Schwenken des rohrförmigen Teils 1 mit angesetztem Narkosemittelbehälter um das im Gelenkstück 5 gegenüber dem Indexstück 8 ausgebildete Drehgelenk läßt sich der Befüll- oder Entleerungsvorgang ausführen.

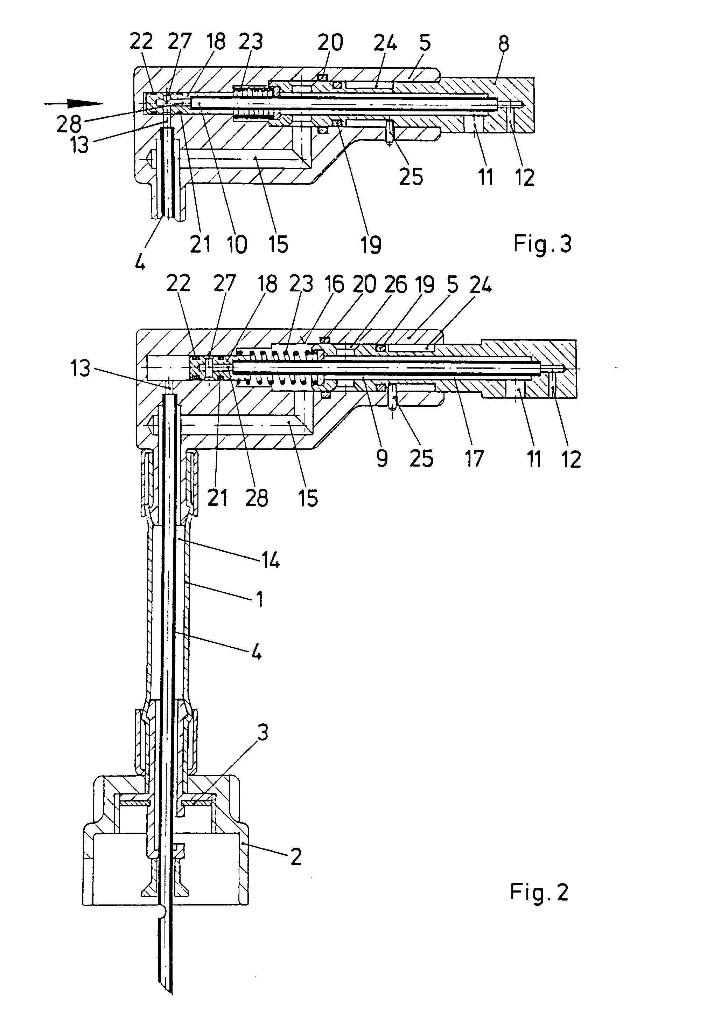

Bei der Ausführungsform nach Fig. 2 und 3 ist im Bereich des zwischen dem Indexstück 8 und dem Gelenkstück 5 gebildeten Drehgelenks eine Absperrvorrichtung integriert, welche in der Absperrlage den Füllkanal und den Belüftungskanal abschließt.

Fig. 2 zeigt die Sicherheitsfüllvorrichtung in der Absperrlage.

Hierzu ist das Indexstück 8 in einer Ausnehmung 16 des Gelenkstücks 5 kolbenartig verschiebbar und über ein zentrales Leitungsstück 17 des Belüftungskanals mit einem gleichfalls kolbenartigen Führungsteil 18 verbunden. Am Indexstück 8 und am kolbenartigen Führungsteil 18 sind jeweils mit diesen Teilen verschiebbare Schnurringdichtungen 19,21,22 vorgesehen. In einer Nut der Ausnehmung 16 liegt eine weitere, feststehende Schnurringdichtung 20.

Das Indexstück 8 liegt gegen eine im Gelenkstück 5 abgestützte Schraubendruckfeder 23 an und wird von dieser in die Absperrlage gedrängt, in der die Leitungsabschnitte 9,10 des Füllkanals und des Belüftungskanals gegenüber den entsprechenden Teilen im rohrförmigen Leitungsstück 1 gesperrt sind. Die Verschiebebewegung des Indexstücks 8 im Gelenkstück 5 wird durch ein in eine hohlzylinderförmige Ausformung 24 eingreifendes Indexteil 25 begrenzt.

Der Leitungsabschnitt 9 des Füllkanals ist mit einer umfangsseitigen Ringnut 26 stellungsunabhängig verbunden. Der Leitungsabschnitt 10 des Belüftungskanals steht über den Leitungsabschnitt 28 mit einer in ähnlicher Weise ausgebildeten umfangsseitigen Nut 27 des Führungsteils 18 in Verbindung.

In der in Fig. 3 dargestellten Gebrauchslage ist das Indexstück 8 in den Verdunster eingeführt, während gleichzeitig auf das Gelenkstück 5 in Pfeilrichtung Druck ausgeübt wird. Dadurch verschiebt sich das Indexstück 8 in dem Gelenkstück 5 unter Mitnahme des Führungsteils 18 in die gezeichnete Stellung, in der die Durchlässe von Füllkanal und Belüftungskanal durch Kommunizieren mit dem gehäusefesten Leitungsabschnitt 15 und der Verbindungsbohrung 13 freigegeben sind.

Die zu den Figuren 2 und 3 nicht erwähnten Teile entsprechen gleichen Bauteilen in Fig. 1.

## Patentansprüche

1. Sicherheitsfüllvorrichtung zum Befüllen und Entleeren eines Narkosemittelverdunsters über einen angeschlossenen Vorratsbehälter, bestehend aus einem bewegbaren, rohrförmigen Leitungsstück (1), welches an seinen Enden mit einem Indexstück (8) zum Eingriff in den Verdunster und mit einem Behälteranschlußstück (2) versehen ist, wobei das rohrförmige Leitungsstück (1) einen vom Füllkanal (14) getrennten Belüftungskanal (4) aufweist, **dadurch gekennzeichnet**, daß am verdunsterseitigen Ende des rohrförmigen Leitungsstücks (1) ein Gelenkstück (5) angeschlossen ist, in dem ein, Leitungsabschnitte (9,10) von Füllkanal und Belüftungskanal aufnehmendes Indexstück (8) drehbar gelagert ist.

2. Füllvorrichtung nach Anspruch 1, **dadurch gekennzeichnet**, daß im Bereich des zwischen Indexstück (8) und Gelenkstück (5) gebildeten Drehgelenks eine Absperrvorrichtung integriert ist, welche in der Absperrlage den Füllkanal und den Belüftungskanal abschließt.

3. Füllvorrichtung nach Anspruch 2, **dadurch gekennzeichnet**, daß die Absperrvorrichtung derart bewegbar ausgebildet ist, daß sich beim Abnehmen des Indexstücks (8) vom Verdunster, die Absperrvorrichtung in die Absperrlage verschiebt.

4. Sicherheitsfüllvorrichtung nach Anspruch 3, **dadurch gekennzeichnet**, daß das Indexstück (8) zwischen der Absperrlage und der Freigabelage verschiebbar ausgebildet ist.

5. Füllvorrichtung nach Anspruch 4, **dadurch gekennzeichnet,** daß das Indexstück (8) in dem Gelenkstück (5) kolbenartig gegen eine Druckfeder (23) verschiebbar gelagert ist und mit dem Füllkanal und dem Belüftungskanal nur in der Freigabelage kommunizierende, drehstellungsunabhängige Leitungsabschnitte (9,10) aufweist.

6. Füllvorrichtung nach Anspruch 5, **dadurch gekennzeichnet**, daß der Belüftungskanal als zentraler Leitungsabschnitt (10)ausgebildet ist, welcher einseitig mit dem Indexstück (8) und mit einem im Gelenkstück (5) bewegbar gelagerten kolbenartigen Führungsteil (18) verbunden ist.

7. Füllvorrichtung nach Anspruch 6, **dadurch gekennzeichnet**, daß die drehstellungsunabhängige Verbindung durch umfangseitige Nutenausnehmungen (26,27) am Indexstück (8) und am Führungsteil (18) gebildet ist.

8. Füllvorrichtung nach Anspruch 1, **dadurch gekennzeichnet**, daß das rohrförmige Leitungsstück (1) aus einem äußeren Füllrohr aus weichmacherfreiem transparentem bzw. transluzentem Kunststoff und einem inneren Belüftungsrohr (4) aus Metall zusammengesetzt ist.

9. Füllvorrichtung nach Anspruch 6, **dadurch gekennzeichnet**, daß zur Abdichtung zwischen dem Gelenkstück (5) und dem in diesem drehbar gelagerten Indexstück (8) mit Führungsteil (18) narkosemittelbeständige Schnurringdichtungen (6;19,20,21,22) vorgesehen sind.

## Claims

1. Safety filling device for filling and emptying an anaesthetic vaporiser via a connected reservoir comprising a movable, tubular conducting piece (1) which is fitted at its ends with an index piece (8) for engagement in the vaporiser and with a container adapter (2), the tubular conducting piece (1) having a ventilation duct (4) separate from the filling duct (14),
characterised in that an articulation piece (5) is attached to the end of the tubular conducting piece (1) nearest the vaporiser by rotatably mounting the index piece (8) which receives filling duct and ventilation duct conducting sections (9, 10).

2. Filling device according to claim 1, characterised in that in the region of the swivel joint formed between the index piece (8) and the articulation piece (5) is incorporated a shut-off device which seals off the filling duct and the ventilation duct in the shut-off position.

3. Filling device according to claim 2, characterised in that the shut-off device is designed to be movable such that when the index piece (8) is removed from the vaporiser the shut-off device moves into the shut-off position.

4. Safety filling device according to claim 3, characterised in that the index piece (8) is adapted to be displaceable between the shut-off position and the throughflow position.

5. Filling device according to claim 4, characterised in that the index piece (8) is mounted in the articulation piece (5) so as to be movable towards a compression spring (23) in the manner of a piston and has conducting sections (9, 10) independent of rotary setting which communicate with the filling duct and with the ventilation duct only in the throughflow position.

6. Filling device according to claim 5, characterised in that the ventilation duct is in the form of a central conducting section (10) which on one side is joined to the index piece (8) and to a piston-type guide member (18) displaceably mounted in the articulation piece (5).

7. Filling device according to claim 6, characterised in that the connection independent of rotary settings is constituted by circumferential grooved recesses (26, 27) on the index piece (8) and on the guide member (18).

8. Filling device according to claim 1, characterised in that the tubular conducting piece (1) comprises an outer filling tube formed of emulsifier-free transparent or translucent plastic and an inner ventilation pipe (4) formed of metal.

9. Filling device according to claim 6, characterised in that anaesthetic-resistant cord ring seals (6; 19, 20, 21, 22) are provided to seal between the articulation piece (5) and the index piece (8) and its guide (18) mounted rotatably in said articulation piece.

## Revendications

1. Dispositif de remplissage de sécurité pour remplir et vidanger un évaporateur d'anesthésique par l'intermédiaire d'un réservoir de stockage branché, constitué d'une conduite mobile en forme de tuyau (1), pourvue, à ses extrémités, d'une pièce formant index (8) pénétrant dans l'évaporateur, et d'un organe de branchement sur le réservoir (2), la conduite en forme de tuyau (1) présentant un canal de ventilation (4) séparé du canal de remplissage (14), caractérisé en ce qu'une pièce articulée (5) est branchée à l'extrémité de la conduite en forme de tuyau (1) située du côté de l'évaporateur, dans laquelle est montée tournante une pièce formant index (8) recevant des parties de conduites (9, 10) du canal de remplissage et du canal de ventilation et pouvant être obturée.

2. Dispositif de remplissage selon la revendication 1, caractérisé en ce que, dans la zone de l'articulation tournante formée entre la pièce formant index (8) et la pièce articulée (5), un dispositif d'arrêt est intégré pour obturer, en position d'arrêt, le canal de remplissage et le canal de ventilation.

3. Dispositif de remplissage selon la revendication 2, caractérisé en ce que le dispositif d'arrêt est conformé de façon mobile de telle sorte que celui-ci se mette en position d'arrêt lorsque la pièce formant index (8) est retirée de l'évaporateur.

4. Dispositif de remplissage de sécurité selon la revendication 3, caractérisé en ce que la pièce formant index (8) peut se déplacer entre la position d'arrêt et la position de déblocage.

5. Dispositif de remplissage selon la revendication 4, caractérisé en ce que la pièce formant index (8), à l'instar d'un piston, peut être montée mobile, à l'encontre d'un ressort de pression (23), dans la pièce articulée (5), et présenter des parties de conduite (9, 10) communiquant, uniquement en position de déblocage, avec le canal de remplissage et avec le canal de ventilation, indépendamment de la position angulaire.

6. Dispositif de remplissage selon la revendication 5, caractérisé en ce que le canal de ventilation est conformé en partie de conduite centrale (10), liée, à une extrémité, à la pièce formant index (8) et, à son autre extrémité, à une pièce de guidage (18) fonctionnant comme un piston et mobile à l'intérieur de la pièce articulée (5).

7. Dispositif de remplissage selon la revendication 6, caractérisé en ce que le raccordement de conduites, indépendant de la position angulaire, est réalisé par des rainures périphériques (26, 27) ménagées sur la pièce formant index (8) et sur la pièce de guidage (18).

8. Dispositif de remplissage selon la revendication 1, caractérisé en ce que la pièce de conduite en forme de tuyau (1) est constituée d'un tuyau de remplissage externe en matière plastique transparente ou translucide, dépourvue de plastifiant, et d'un tuyau de ventilation (4) interne en métal.

9. Dispositif de remplissage selon la revendication 6, caractérisé en ce que, pour assurer l'étanchéité entre la pièce articulée (5) et la pièce formant index (8) montée tournante dans celle-ci et pourvue d'une partie de guidage (18), sont prévus des cordons annulaires d'étanchéité (6 ; 19, 20, 21, 22) résistant aux anesthésiques.
